(19) Europäisches Patentamt
European Patent Office
Office européen des brevets

(11) Publication number: **0 202 758 B1**

(12) **EUROPEAN PATENT SPECIFICATION**

(45) Date of publication of patent specification: **25.09.91**

(51) Int. Cl.$^5$: **C12Q 1/68**, C07H 21/00

(21) Application number: **86302750.4**

(22) Date of filing: **14.04.86**

(54) **Hybridization method and probes therefor.**

(30) Priority: **17.04.85 GB 8509880**

(43) Date of publication of application:
**26.11.86 Bulletin 86/48**

(45) Publication of the grant of the patent:
**25.09.91 Bulletin 91/39**

(84) Designated Contracting States:
**AT BE CH DE FR GB IT LI LU NL SE**

(56) References cited:

| | |
|---|---|
| EP-A- 0 117 440 | EP-A- 0 119 448 |
| EP-A- 0 138 357 | EP-A- 0 154 505 |
| WO-A-84/03285 | WO-A-84/03520 |
| FR-A- 2 556 726 | GB-A- 2 019 408 |

**PROCEEDINGS OF THE NATIONAL ACADEMY OF SCIENCES (USA), vol. 80, January 1983, pages 278-282; B.J. CONNER et al.: "Detection of sickle cell betaS-globin allele by hybridization with synthetic oligonucleotides"**

(73) Proprietor: **IMPERIAL CHEMICAL INDUSTRIES PLC**
**Imperial Chemical House, Millbank**
**London SW1P 3JF(GB)**

(72) Inventor: **Carr, Francis Joseph**
**16 Snabwood Close**
**Little Neston South Wirral L64 0UP(GB)**
Inventor: **Edge, Michael Derek**
**6 Tudor Way**
**Congleton Cheshire(GB)**

(74) Representative: **Mack, John Richard et al**
**Imperial Chemical Industries PLC Legal Department: Patents PO Box 6 Bessemer Road**
**Welwyn Garden City Hertfordshire AL7 1HD(GB)**

**EP 0 202 758 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a method suitable for discriminating between two complementary nucleotide sequences which may differ by as little as a single nucleotide, nucleic acid hybridisation probes therefor and kits for use in such a method.

The use of labelled polynucleotide probes is well known for a wide range of applications as described in, for example, Lewin, Science 221:1167 (1983) and Klausner et al, Bio/Technology, 1: 471 (1983). Labelled oligonucleotide probes are of particular interest as diagnostic tools for clinical and research uses.

Conventional radiolabelled probes are efficient and sensitive, but are associated with several problems which mitigate against routine use for screening for example in clinical laboratories. Thus radiolabelled probes are potentially hazardous and pose problems of disposal. Furthermore radiolabelled probes are often unstable and have a limited shelf-life as a result of the relatively short half life of the radioactive materials used as labels, especially $^{32}P$. Moreover autoradiographic detection can be time consuming and the handling of radiolabelled probes requires personnel with the appropriate safety training.

There is therefore a desire to use non-radioactive methods of labelling probes and certain such methods have been described in the literature for example D.C. Ward at the 1981 ICN-UCLA Symposium held in Keystone, Colorado on March 15-20, and published in Developmental Biology Using Purified Genes 1981 XXIII, 1981 pages 647-658 Academic Press, Editor Donald D Brown et al; and A D B Malcolm et al, Abstracts of the 604th Biochemical Society Meeting, Cambridge, England (meeting of 1 July 1983). Probes labelled with biotin have also been widely described in the literature.

The present invention however is particularly concerned with the use of non-radioactively labelled probes for use in hybridization analysis where it is necessary to discriminate between two complementary nucleotide sequences differing by relatively few nucleotides. It is well documented (e.g. by J W Szostok et al., Methods in Enzymology, Volume 68, p419-428. Academic Press) that the stability of hybrids of oligonucleotides with complementary nucleic acid sequences is sensitive to mismatched base pairs in the hybrid. These authors demonstrated that most oligonucleotide hybrids with single mismatches in base pairing are less stable to temperature than hybrids of the same oligonucleotides with correct base pairing. Thus subtle differences in oligonucleotide sequence complementarity can give rise to subtle differences in hybrid destabilisation which can be detected in practice by incubating matched and mismatched oligonucleotide hybrids to a specific temperature in aqueous solutions. It will therefore be appreciated that the present invention is concerned with non-radioactively labelled probes which have sufficient selectivity to discriminate between a complementary sequence and a sequence with as little as 1-3 non-complementary nucleotides. Because of the relatively small destabilisation effect of base pair mismatches on oligonucleotide hybrids, it would be expected that labelling of an oligonucleotide probe with anything but a low molecular weight species would alter the hybridization characteristics of the probe such that the desired hybridisation selectivity of the probe would be lost as a result of the presence of the label. Recently A Chollet and E H Kawashima in Nucleic Acids Research Vol 13 No. 5 1985 have demonstrated that a biotinylated probe may be used to detect a single A/C mismatch whilst recognizing that even the low molecular weight species, biotin, may alter the hybridisation characteristics of the oligonucleotide probe.

The belief that even the attachment of low molecular weight labels to oligonucleotide probes may adversely affect their ability to distinguish complementary and mismatched sequences involves the practical disadvantage that the detection system can only be attached to the probe after hybridisation. The operator of the probe is thus faced with conducting further reaction steps after the hybridisation which necessarily are time consuming, costly and conducive to operator error.

The present invention is based on the surprising discovery that high molecular weight species may be attached to oligonucleotide probes prior to hybridisation without markedly altering the hybridisation selectivity of the probe. Thus for example a high molecular weight detection system may be attached to an oligonucleotide probe prior to hybridisation thus obviating the need for a series of reaction steps after hybridisation.

Thus according to one feature of the present invention there is provided a method for discriminating between two complementary nucleotide sequences differing by as little as 1-3 nucleotides which comprises:-

(a) under conditions which promote or favour hybridisation between two complementary oligonucleotide strands, contacting the nucleotide sequence (after appropriate treatment to render it single stranded) to be identified or determined with an oligonucleotide-signalling complex probe comprising a single stranded oligonucleotide of 8 to 30 nucleotide units in length and having covalently attached thereto, at a sterically tolerant site, a residue having a molecular weight of at least 1,000 daltons, which residue is capable of producing a non-radioisotopic signal, the oligonucleotide in the oligonucleotide-signal complex

having a defined sequence which is complementary to a sequence to be identified or determined but which is not complementary to an alternative sequence;

(b) separating the probe:test nucleic acid hybrid from the unhybridised probe; and

(c) producing or observing the signal resulting from the hybridised oligonucleotide-signalling complex.

The residue which is capable of producing a non-radioisotopic signal advantageously has a molecular weight of at least 5000 daltons and preferably of at least 20,000 daltons. Whilst we do not wish to be bound by theoretical considerations we have determined that in general a residue having a molecular weight as high as about 2 million daltons may for example be used with advantage in the method of the present invention. Preferably the residue will comprise a proteinaceous moiety.

The method of the present invention is preferably effective to discriminate between two complementary nucleotide sequences differing by as little as only a single nucleotide.

The method of the present invention is especially useful for detecting the presence of point mutations in genes, particularly where these point mutations are responsible for, or are associated with, particular disease states. Examples of such disease states are phenylketonuria, alpha$_1$-antitrypsin deficiency, alpha- and beta-thalassaemia and sickle cell anaemia. In addition the method of the present invention is useful for detecting the presence of polymorphisms in mammalian genomes, some of which are often associated with particular disease states. The method of the present invention is however useful in a number of other areas. For example, it is useful for testing the accuracy of a nucleotide sequence in a gene cloned into a plasmid or phage vector.

The oligonucleotide-signalling complex is capable of forming a hybrid with the complementary test nucleic acid sequence under such conditions that one (or more) single base change(s) in this complementary sequence reduces or precludes hybrid formation.

The nucleic acids contained in the probe may be ribonucleotides, or deoxyribonucleotides, or a mixture of these two.

The single stranded oligonucleotide is preferably composed of 12 to 25 nucleotide units, and most preferably of 17 to 20 nucleotide units when the test sample contains mammalian nucleic acids.

The residue which is capable of producing a non-radioisotopic signal may be covalently attached to a base, a sugar or preferably a phosphate of the oligonucleotide. Such attachment is preferably on a terminal base, sugar or phosphate, either at the 3' or 5' end of the oligonucleotide. When the attachment is via phosphate it is preferably at the 5' end.

When the covalent attachment is on a base of the oligonucleotide, it is particularly important that the attachment is located at a sterically tolerant site, that is a position on the base at which modification can be effected by attachment thereto of a substituent group without causing interference with the ability of the modified oligonucleotide to hybridise with a fully complementary sequence. Generally, substitution on any hetero atom (nitrogen or oxygen) of the base is less desirable. Specific sites that are less desirable are $N^1$ and $N^6$ of adenine ($N^1$ being less desirable than $N^6$), $N^1$, $N^2$ and $O^6$ of guanine and $N^3$ and $N^4$ of cytosine. Preferred sites of covalent attachment are $C^5$ of cytosine and uracil and $C^8$ of adenine and guanine.

Methods of introducing a covalently-bonded group in a base of an oligonucleotide are known from, for example, European Patent Publications 63879 (Yale University) and 97373 (Enzo Biochem.) and PCT Patent Publication WO84/03285 (Molecular Biosystems).

Methods of introducing a covalently-bonded group in a phosphate of an oligonucleotide are known from, for example, European Patent Publications 97373 (Enzo) and 119448 (Wakunaga Seiyaku Kabushiki Kaisha) and T Kempe et al, Nucleic Acids Research, 1985, 13, page 45. .

The introduction of a covalently bonded group in a sugar residue of an oligonucleotide may, for example, be carried out as follows. The group may, for example, be bonded via a hydroxy group in the sugar, for example by an ether or ester link. This reaction may be carried out by any standard method known in the art, for example that described by J Smrt, Coll. Czech. Chem. Comm., 1968, 33, 1462 and A. Stuart et al., J. Amer. Chem. Soc., 1963, 85, 2346, and J Biol. Chem., 1964, 239, 3885. When the sugar is a deoxyribose a free hydroxy group is available only when the nucleotide containing this sugar is at the 3' or 5' end of the oligomer. When the sugar is a ribose a free hydroxy group is also available at a 2' position and the nucleotide containing this sugar can occupy any position in the oligomer. Alternatively, the group may be bonded via an aldehyde function, obtained by cleaving a diol in a ribose of an oligonucleotide. Such a diol is only available when the nucleotide containing the ribose occupies the 3' end of the oligomer. Such a reaction is described by F Hausske and F Cramer, Methods in Enzymology, 1979, 59, 172-181.

It will be understood that the residue which is capable of producing a non-radioisotopic signal may itself be capable of signalling or may be capable of producing a signal by interaction with an appropriate agent according to methods known per se. The residue is thus defined herein as consisting of a spacer group linked to a detection system, the residue having a molecular weight of at least 1000 daltons. Preferably the

detection system will comprise a proteinaceous moiety.

The detection system comprises a signalling moiety either alone or attached to a linkage for attachment to the spacer group. Thus the signalling moiety may either be directly attached to the spacer group by a direct covalent bond or may be attached to the spacer group via a linkage.

Thus for example where a proteinaceous moiety is present it may be present in the signalling moiety of the detection system or, where a linkage is present, in the linkage between the signalling moiety and the spacer group or in both such linkage and signalling moiety. The spacer group may thus if desired be linked to the signalling moiety of the complex by a direct covalent link in which case the signalling moiety will preferably comprise a proteinaceous moiety such as a system for producing an enzymatically-activated production of a colour change.

In a further embodiment the spacer group may if desired be linked to the signalling moiety of the detection system by a proteinaceous moiety containing specific binding pair, for example a protein-ligand or antigen-antibody interaction for example an avidin-biotin or dinitrophenyl-antidinitrophenyl antibody interaction. If desired the signalling moiety may also comprise a proteinaceous moiety such as defined above in relation to the signalling moiety.

In a still further embodiment the spacer group may for example simply be linked to a proteinaceous moiety containing specific binding pair or the proteinaceous partner of such a pair.

A preferred signalling part of the residue incorporates a system for producing an enzymatically-activated production of colour change. Preferred enzyme systems involve alkaline phosphatase (AP), acid phosphatase, beta galactosidase, luciferase or horseradish peroxidase. Such enzyme systems are not themselves capable of signalling, but are capable of producing a signal in the presence of an appropriate substrate according to methods known per se. The signalling part of the covalently-attached residue may operate according to any conventional technique such as for example by luminescence, fluorescence or by means of colour.

In use it will generally be necessary for the nucleic acid probe to detect minute amounts of the fully complementary oligonucleotide sequence. In such circumstances it will be advantageous to incorporate within the probe a means of amplifying the signal. The amplification can be carried out by known techniques, for example using one or more of the systems described in European Patent Publications 27036 (Self), 49606 (Self), 58539 (Self), and 60123 (Self).

The means employed to produce or observe the signal will depend on the signalling mechanism. Thus, for example, where the signal incorporates an enzymatically-activated colour or colour change it will generally be necessary to supply the enzyme with a substrate.

In employing the method of the invention it will generally be advantageous to employ a probe which the point mutation has occurred at or near the middle of the sequence. In this way the differentiation between normal sequence and the abnormal altered sequence will tend towards the optimal. It will be understood by those skilled in the art that discrimination of nucleotide sequences where a point mutation occurs towards a region opposite the terminus of an oligonucleotide probe is, in practice, more difficult as exemplified by Szostak et al., Methods in Enzymology, Vol 68., p. 419 (Academic Press).

The method of the invention will be carried out using standard techniques and reagents, for example, employing the "Souther blot", "dot blot" or "Sandwich hybridisation" techniques.

The oligonucleotide signalling complex comprises a single stranded oligonucleotide of 8 to 30 nucleotide units in length and having covalently attached thereto at a sterically tolerant site, via a spacer group, either a residue comprising a specific binding pair optionally carrying an enzyme label, said residue having a molecular weight of at least 1000 daltons or an enzyme label with the proviso that where an enzyme label is linked to the oligonucleotide via a spacer group, the linkage of the spacer group to the oligonucleotide is via the terminal base, sugar or preferably phosphate.

On the basis of experiments which we have conducted we have concluded that linkage of the spacer group to the terminal base, sugar or phosphate alone has negligible effect on the melting temperature of the oligonucleotide signalling complex. This is in contrast to a reduction in melting temperature which has been reported in the literature for substitutions at positions other than at the terminal base, sugar or phosphate alone.

The spacer group is preferably linked to the oligonucleotide via phosphate at the 5'-end.

Where the enzyme label is directly linked to the oligonucleotide via a spacer group the spacer group is conveniently of formula A in which n is 2 to 16, preferably 6, a is 0-8, b is 0-8 and R represents a direct covalent link or a cyclohex-4-yl, 2-phenylene, 3-phenylene or 4-phenylene group optionally substituted by 1 to 3 radicals selected from $C_{1-6}$ alkoxy e.g. methoxy and di($C_{1-6}$ alkyl)amino e.g. dimethylamino. Conveniently a is 0, b is 0 and R is 3-phenylene.

The oligonucleotide of the said oligonucleotide-signalling complex preferably has a sequence complementary to a nucleotide sequence associated with a disease state or with the corresponding normal sequence.

The specific binding pair is for example an immunological pair, conveniently an antigen/antibody interaction for example a dinitrophenyl-antidinitrophenyl antibody interaction or a protein-ligand interaction preferably avidin-biotin.

Preferably a system for producing an enzymatically-activated production of colour change or enzyme label is covalently attached to the said specific binding pair. Preferred enzyme systems include alkaline phosphatase, acid phosphatase, beta-galactosidase, luciferase or horseradish peroxidase. Such enzyme systems are not themselves capable of signalling, but are capable of producing a signal in the presence of an appropriate substrate according to methods known per se. The signalling part of the covalently-attached residue may operate according to any conventional technique such as for example by luminescence, fluorescence or by means of colour, provided that a proteinaceous moiety is present as part of the detection system.

It will generally be advantageous to incorporate within the probe a means for amplifying the signal. Such amplification can be effected by known techniques for example using one or more of the systems described in European Patent Publications 27036, 49606, 58539 and 60123.

Preferably the obligonucleotide probes of the present invention possess the formula B wherein A represents avidin or an avidin-enzyme moiety m is 4 or 5, p is 0 to 16 and X is a direct link, -O-P(0)(OH)-O-, -S-, -O-, -CONH-, CONHCO, or less preferably N-$R^8$ (wherein $R^8$ represents a straight chain or branched $C_{1-10}$ alkyl group). Normally when X is -CONH- or -CONHCO-then m is 4. X is preferably a direct link or -O-P(0)(OH)-O-.

If desired the group A in which A is an avidin-enzyme moiety may additionally carry a substrate for the enzyme.

In particular the oligonucleotide probe is especially a probe of formula B (i) in which X is a direct link, m is 5 and p is 0 or (ii) in which X is -O-P(0)(OH)-O-, m is 5 and p is 8.

According to a further feature of the present invention there is provided a process for the preparation of an oligonucleotide signalling complex as hereinbefore defined wherein the residue comprises a specific binding pair optionally carrying an enzyme label, said residue having a molecular weight of at least 1000 daltons, the specific binding pair being covalently attached via a spacer group to the single stranded oligonucleotide, which process comprises reacting one member of said specific binding pair optionally carrying an enzyme label with an oligonucleotide complex comprising a single stranded oligonucleotide of 8 to 30 nucleotide units in length and having covalently attached thereto, at a sterically tolerant site, the other member of said specific binding pair via a spacer group, the reactants being selected such that the residue in the complex obtained has a molecular weight of at least 1000 daltons. Preferably at least one of the members of the specific binding pair comprises a proteinaceous moiety.

The oligonucleotide complex containing only one member of said specific binding pair is conveniently prepared by reacting a protected oligonucleotide of 8 to 30 nucleotide units with a free OH moiety of an otherwise protected phosphate of one member of said specific binding pair and deprotecting the product thereby formed.

The phosphate of one member of the specific binding pair is preferably prepared by reacting an -OH group of the said member with a phosphate of the formula $R^1$-O-P(O)($R^2$)$R^3$ in which $R^1$ represents a protecting group for the phosphate and $R^2$ and $R^3$, which may be the same or different each represents a leaving group, whereby a phosphate of one member of the specific binding pair is obtained in which the phosphate carries a single phosphate protecting group.

It is essentially preferred to prepare an oligonucleotide signalling complex as hereinbefore defined in which the residue comprises a specific binding pair optionally carrying an enzyme label by first preparing the oligonucleotide complex containing only one member of the specific binding pair using the phosphate of the formula $R^1$-O-P(O)($R^2$)$R^3$ as hereinbefore defined and then reacting the complex obtained with the other member of the specific binding pair optionally carrying an enzyme label.

Preferably the reaction is effected in the presence of an agent effective to introduce a phosphate protecting group whereby to isolate a phosphate of one member of the specific binding pair, which phosphate carries two phosphate protecting groups and subsequently removing one of said phosphate protecting groups. Such a process provides an advantageous method of purifying the product of the phosphorylation process.

The above-mentioned phosphate is preferably 2-chlorophenyl di(1,2,4-triazole)phosphate and the phosphate protecting group is preferably introduced in the presence of a base such as pyridine.

According to a further feature of the present invention there is provided a process for the preparation of an oligonucleotide signalling complex as hereinbefore defined in which an enzyme label is covalently

attached via a spacer group of the formula A to the single stranded oligonucleotide, which process comprises reacting a compound of formula C (in which E represents an enzyme such as alkaline phosphatase or horseradish peroxidase) with a compound of formula D in which n is as hereinbefore defined.

The compound of formula C is preferably prepared by reacting an amino group of the enzyme with a compound of formula F in which Y represents an activated ester and R, a and b are as hereinbefore defined. Preferably Y represents a group of the formula G in which $R^7$ represents hydrogen or a sulphonic acid salt preferably the sodium salt. Preferably a compound of formula F is used in which $R^1$ is hydrogen, a is 0, b is 0 and R is the 3-phenylene moiety (m-phenylene). The compound of formula D in which n is as hereinbefore defined is preferably prepared by deprotection, for example by hydrolysis, of a corresponding compound of formula E. The compound of Formula E is preferably prepared by reacting the oligonucleotide in protected form, conveniently protected on a polymer support with a compound of formula H in which n is as hereinbefore defined, $R^4$ and $R^5$, which may be the same or different, each represents a $C_{1-10}$ straight chain or branched alkyl group, preferably methyl or isopropyl or $R^4$ and $R^5$ together with the nitrogen atom therebetween represents a morpholine ring and $R^6$ represents a protecting group suitable for a phosphate, preferably a methyl group.

The compound of formula H is preferably prepared by reacting a compound of formula J in which n is as hereinbefore defined with a compound of formula K in which $R^4$, $R^5$ and $R^6$ are as hereinbefore defined and Hal represents a halogen atom preferably chlorine. The compound of formula K is preferably chloro N,N-diisopropylamino methoxy phosphine.

When the oligonucleotide-signalling complex and method of the invention are employed in the detection of a point mutation in a gene associated with a disease state, it will generally be advantageous to incorporate the oligonucleotide-signal complex in a diagnostic kit and this kit is therefore regarded as a further feature of the invention.

Thus according to a further feature of the present invention there is provided a diagnostic kit for discriminating between two complementary nucleotide sequences differing by as little as a single nucleotide, one of the said sequences being associated with a disease state and the other of said sequences being the corresponding normal sequence, which kit comprises at least one oligonucleotide-signalling complex probe comprising a single stranded oligonucleotide of 8 to 30 nucleotide units in length, said oligonucleotide having a sequence complementary to a sequence associated with a disease state or the corresponding normal sequence, said oligonucleotide having covalently attached thereto, at a sterically tolerant site, a residue having a molecular weight of at least 1000 daltons, which residue is capable of producing non-radioisotopic signal.

The kit will preferably comprise 1) at least one oligonucleotide signalling complex probe comprising a single stranded oligonucleotide having a sequence complementary to a sequence associated with a disease state and 2) at least one oligonucleotide signalling complex probe comprising a single stranded oligonucleotide having a sequence complementary to the corresponding normal sequence which would be present in a human not subject to the said disease state.

It will be appreciated in this regard that certain disease states are characterised by point mutations which appear in different regions of the human DNA genome. In such a case it would be important to determine that each relevant point mutation was present or absent and thus more than one oligonucleotide signalling complex probe would be employed, one probe to detect the presence or absence of each point mutation. It would also be advantageous to include in the kit the probe for the corresponding normal or abnormal sequence.

The kit will also preferably comprise appropriate buffer solutions and/or washing solutions and will contain written or printed instructions for use of the kit according to the method of the present invention.

In the accompanying drawings:

Figure 1 shows the hybridisation signal observed for the immobilised pIF-25 alpha$_2$ DNA [row B, 3 ug (nearest B), 1 ug (remote from B)] and the fact that no signal was observable in respect of the mutant pIF25-15 alpha$_2$ plasmid DNA [row A, 3ug (column nearest B) 1 ug (column remote from B)]. (See Example 7).

Figure 2 shows discrimination by the oligonucleotide-alkaline phosphatase complex between 300 ng. (column nearest A) and 30 ng (column remote from B) of the immobilised pIF55 alpha$_2$ plasmid DNA (row A) and the pIF55-F6 alpha$_2$ derivative plasmid DNA (row B). The pIF55 alpha$_2$ plasmid DNA (row C) and its derivative pIF55-F6 alpha$_2$ (row D) were mock hybridised without the oligonucleotide complex, but with alkaline phosphatase. The absence of signals in rows C and D rules out the presence of artefacts due to direct binding of alkaline phosphatase (See Example 8).

Figure 3 shows discrimination by the oligonucleotide-AP complex between 300 ng (column nearest to A)

and 30 ng (column remote from A) of pIF55 $alpha_2$ (row A) and pIF55-F6 $alpha_2$ (row B). The signal intensity produced by the probe being stronger for the homologous plasmid pIF55-F6 $alpha_2$ (See Example 9).

Figure 4 shows much stronger hybridisation of the probe to (left to right) 300 ng, 30 ng and 3 ng of the homologous plasmid pIF25 $alpha_2$ (row A) than to 300 ng, 30 ng and 3 ng of the plasmid pIF25-I4 $alpha_2$ (row B) (See Example 10).

Figure 5 shows that after Southern transfer 300 ng of linearised pIF55-F6 $alpha_2$ would be detected as a single band either in the presence (lane 1) or absence (lane 5) of an excess of human DNA. Under the same conditions, 300 ng of linearised pIF55-F6 $alpha_2$ DNA was not detectable in the presence (lane 2) or absence (lane 6) of Daudi DNA illustrating discrimination by an $alpha_2$ 55 oligonucleotide-AP complex between pIF$alpha_2$ and pIF55-F6 $alpha_2$ DNA.

The invention is illustrated, but not limited by the following Examples. In the Examples, unless otherwise stated, the solutions are aqueous and the % values are w/v.

The constitution of various reagents is as follows:-

PBS 150mM NaCl + 10mM sodium phosphate at pH7.4
SSC 0.15M NaCl + 0.015M sodium citrate
Denhardt's reagent 0.02% BSA
0.02% Ficol 400,000
0.02% PVP

The following contractions are used:-

MeOH methanol
EtOAc ethyl acetate
DMF dimethylformamide
DNA deoxyribonucleic acid
tRNA transfer ribonucleic acid
HRP horseradish peroxidase
PBA phosphate buffered saline
SDS sodium dodecyl sulphate
2 × SSC double concentration SSC
0.5 × SSC half concentration SSC
BSA bovine serum albumin
PVP polyvinyl pyrrolidone
HEPES (N-2-hydroxyethyl-N'-2-ethane sulphonic acid)
Tris tris(hydroxymethyl)aminomethane
AP alkaline phosphatase

The following are trade marks

| | | |
|---|---|---|
| Fractosil | Partisil 10-SAX | C18-u Bondapak |
| Triton-X-100 | NENSORB | Sephadex G-25 |
| Tween | Millex | Sephadex G-50 |
| Vectastain | Centricon | |
| Nonidet P40 | Ampak | |
| Ficol 400,000 | Core Buffer | |

Example 1

A molecule of biotin was attached to a 15 base long oligonucleotide $alpha_2$-55 with the nucleotide sequence of (6) by the following method.

Preparation 1

A solution of phosphorylating agent prepared from 2-chlorophenylphosphorodichloridate (0.32 ml.) and 1,2,4-triazole (700 mg.) in pyridine (3 ml.) was added to anhydrous biotinol (1) (230 mg.) and the mixture

stirred at 20°C for 30 minutes. 2-Hydroxypropionitrile (0.68 ml.) was then added and stirring was continued for a further 1.5 hours. The mixture was evaporated and the residue partitioned between chloroform (20 ml.) and brine (20 ml.). The chloroform extract was dried ($MgSO_4$) and evaporated. The residue was fractionated on a column of silica gel (50 g.) using $CHCl_3$:MeOH (19:1v/v; 100 ml.) as the first eluant, followed by $CHCl_3$:MeOH (9:1 v/v). After evaporation of solvents from combined appropriate fractions (as indicated by thin layer chromatography analysis on silica gel using $CHCl_3$:MeOH 4:1 v/v as eluant and iodine vapour for detection), there was thus obtained biotinyl 2-chlorophenyl 2-cyanoethyl phosphate (2) (0.32 g., 67%) as an oil.

Preparation 2

A solution of biotinyl 2-chlorophenyl 2-cyanoethyl phosphate (2) (230 mg.) in a mixture of pyridine (3 ml.), triethylamine (1 ml.) and water (0.5 ml.) was allowed to stand at 20°C for 1 hour, then evaporated. The residue was extracted with ether (6 x 20 ml.) and dried by azeotropic distillation with anhydrous pyridine at reduced pressure to give the triethylammonium salt of biotinyl 2-chlorophenyl phosphate.

Preparation 3

A sample (10 mg.) of a fully-protected oligodeoxyribonucleotide sequence, known as sequence 55 in an interferon-alpha$_2$ gene synthesis described by M. D. Edge et al (Nucleic Acids Res., 1983 11, 6419-6435), attached to a polydimethylacrylamide resin was swollen in DMF (15 minutes at 20°C) in a glass column (6.5 x 50 mm) fitted with a variable-length plunger and glass wool frit (see M. J. Gait et al J. Chem. Soc., Chem. Commun., 1982, 37-40). The top of the column was connected via polytetrafluoroethylene tubing to a six-way rotary valve which enabled solvents to be selected and passed through the column, under nitrogen pressure (10 psi), at a flow rate of 2-3 ml./ minute. The column was washed successively with pyridine, $CHCl_3$:MeOH (7:3 v/v), (2 minutes each), 5% benzenesulphonic acid in $CHCl_3$:MeOH (7:3 v/v) (40 seconds) to remove the 5'-dimethoxytrityl group, DMF (2 minutes) and pyridine (2 minutes). The resin was dried by passing a stream of nitrogen through the column at 50°C.

After disconnecting the column from the solvent delivery system and removing the column top, a solution of biotinyl 2-chlorophenyl phosphate triethylammonium salt (23 mg.) and (1-mesitylene)-3-nitro-1,2,4-triazole (50 mg.) in anhydrous pyridine (0.3 ml.) was added to the dried resin described above. The column top was replaced and the column placed in an oven at 45°C for 45 minutes. (Alternatively, the reaction can be performed at 20°C for 1.5 hours.) The column was re-attached to the solvent delivery system then the resin washed with pyridine (2 minutes), DMF (2 minutes), $CHCl_3$:MeOH (7:3 v/v) (2 minutes), ether (2 minutes) and finally dried in a stream of nitrogen to give the biotinylated fully-protected oligonucleotide sequence (4) attached to the polydimethylacrylamide resin.

The biotinylated oligonucleotide sequence was cleaved from the solid support and deprotected by a standard procedure as described by M.D. Edge et al (Nature, 1981, 292, 756-762), except that treatment with 80% acetic acid was not required, then purified by a two-stage high-performance liquid chromatographic (hplc) procedure as described by C. R. Newton et al, (Anal. Biochem., 1983 129, 22-30) to give the biotinylated oligodeoxyribonucleotide sequence (5). The first hplc on Partisil 10-SAX ion exchange resin (using buffer system IV or VI from Newton et al.) gave the product, biotinylated-alpha$_2$55 oligonucleotide (5), as the most retained peak with a retention time of 42 minutes (buffer system IV). The parent oligonucleotide sequence (alpha$_2$-55) (6) had a retention time of 38 minutes when analysed under identical conditions.

In order to establish that a biotin residue had been attached to the oligonucleotide sequence, an aqueous solution of the product (1.6 O.D.$_{260}$ units in 2 ml. of 1 x PBS, 0.1% Tween 20) was added to a column (0.25 ml.) of avidin-agarose (Sigma Chemical Company Ltd) containing 8.3 units of avidin. The column was washed with 1 x PBS, 0.1% Tween and fractions (1 ml.) were collected. At least 85% of the u.v. absorbing material was retained by the column. In contrast, when a sample of $^{32}$P-labelled oligonucleotide sequence (alpha$_2$-55) was added to a similar column, all the radioactivity passed through the column in the first 2 ml. of eluant.

A complex of avidin and HRP was attached to the biotinylated oligonucleotide using a Vectastain ABC kit (Vector Laboratories, Burlingame, California 94010, USA). One drop of Vectastain reagent A was added to 2 ug. of biotinylated oligonucleotide in 1 ml. of PBS containing 0.1% Tween 20 (Sigma Chemical Company). After mixing, one drop of Vectastain reagent B was added, mixed and the mixture allowed to stand at room temperature for 30 minutes.

Plasmids for analysis contained either the interferon-alpha$_2$ gene nucleotide sequence 3'

TTCTTTATGTCGGGC 5' (M. D. Edge et al. Nucleic Acids Res., 1983, 11, 6419) homologous to the sequence of oligonucleotide $alpha_2$-55 (6) (the plasmid being hereinafter referred to as pIF55 $alpha_2$), or the sequence 3' TTCTTTATGTCGACG 5' which has a terminal three base non-homologous mismatch region (underlined) within the oligonucleotide. This latter plasmid, which is hereinafter referred to as pIF55-F6 $alpha_2$, was obtained as follows:-

A gene was prepared from synthetic oligonucleotides essentially as described by M. D. Edge et al (Nucleic Acids Research, 1983, 11, 6419-6435) for synthesis of an interferon-$alpha_2$ gene except that ologonucleotides 1, 2, 55, 56 and 57 were replaced by the sequences 1 Taq, 2 Taq, 55-F6, 56-F6 and 57-F6 respectively.

1 Taq 5' CGACGATGTGTGAT
2 Taq 5' CGGCAGATCACACATCGT
55-F6 5' AAGAAATACAGCTGC
56-F6 5' CCGGCTTGGCAGCTG
57-F6 5' CAAGCCGGGCAGCTG

The synthetic gene was cloned between the ClaI and SalI sites of the expression plasmid pSTP1 (Windass et al, Nucleic Acids Res., 1982 10, 6639-6657) using standard procedures (Maniatis et al, Molecular Cloning; A Laboratory Manual: Cold Spring Harbor Laboratory, 1982).

Serially diluted samples of plasmid DNA (1 ug maximum) were "dot-blotted" or "slot-blotted" onto a nitrocellulose filter using equipment and instructions supplied by Schleicher and Schuell, Keene, New Hampshire, USA. The filter was air-dried and baked in vacuo for 2 hours at 80° C. Filters were then wetted in 2 × SSC and prehybridised for 2 hours at 68° C in a mixture containing 5 × SSC, 5 × Denhardt's reagent, 1% glycine, 0.1% SDS, 50 mM sodium phosphate (pH 7.0) and 100 ug/ml. denatured herring sperm DNA. Filters were hybridised for 16 hours at room temperature using 1 ml. of the oligonucleotide-protein (avidin + HRP) complex added to 5 ml. of a mixture containing 5 × SSC, 0.5 % Nonidet P40 and 250 ug/ml. tRNA. Filters were then washed twice for 5 minutes at room temperature in a mixture containing 6 × SSC, 0.06% sodium pyrophosphate and 20 mM sodium phosphate, pH 7.0. Filters were further washed in 6 × SSC, 0.06% sodium pyrophosphate and 20mM sodium phosphate twice for 2 minutes at 37° C and once for 2 minutes at 40° C, a temperature at which the oligonucleotide hybridised with three terminal mismatches to the plasmid pIF55-F6 $alpha_2$ was washed off the filter to a much greater extent than the oligonucleotide hybridised to pIF55 $alpha_2$ DNA.

Hybridised oligonucleotide-protein complexes were visualized as follows. Filters were soaked for 30 minutes at 37° C in PBS containing 2% BSA and 0.1% Triton-X-100 and then for 30 minutes at 37° C in 10 ml. PBS/0.1% Tween 20. Filters were then washed at room temperature for 5 minutes three times and twice respectively in each of the following solutions:

| | | |
|---|---|---|
| Solution 1:- | 0.5 M | NaCl |
| | 10 mM | sodium phosphate pH 6.5 |
| | 0.1% | BSA |
| | 0.05% | Tween 20 |
| Solution 2:- | 2 x SSC | |
| | 0.1% w/v | BSA |
| | 0.05% | Tween 20 |

The HRP substrate was prepared by dissolving 5 mg. of diaminobenzidene in 10 ml. of 10mM Tris, pH 7.5. A solution of 200 ul. of 1% $CoCl_2$ was added, mixed well and the mixture left on ice in the dark for 10 minutes. Following this 10 min incubation, 15 ul of 30% $H_2O_2$ was added. Finally filters were placed in the substrate mixture resulting in visual localisation of hybridised oligonucleotide-protein complexes as dark brown deposits. A hybridisation signal corresponding to a minimum of 300 ng of pIF55 $alpha_2$ DNA was detected while signal was absent from immobilised "dots" of pIF55-F6 $alpha_2$ DNA where three base pairs at one end of the oligonucleotide were non-complementary to the corresponding sequence. This discrimination forms the basis for a test to distinguish alterations in a nucleotide sequence homologous to an oligonucleotide probe.

Analysis of hybrids detected with complexes of avidin and biotinylated HRP indicates that the oligonucleotide-protein complexes so formed include up to 30 molecules of biotinylated HRP and 10 molecules of avidin giving a maximum total molecular weight of about 1,880,000 daltons. Thus very large masses of protein complexed to oligonucleotide probes as exemplified do not interfere with the hydridisation selectivity of the oligonucleotide-protein complex.

## Example 2

The procedure described in Example 1 was followed except that the biotinylated oligonucleotide was bound with avidin alone for hybridisation. This avidin binding was performed as described in Example 1 except that Vectastain reagent B (ie HRP) addition was omitted. Following hybridisation and washing at temperatures to distinguish mismatches from homologous hybrids, filters were soaked in BPS, 2% BSA, 0.1% Triton X-100 for 30 minutes at 37°C as described in Example 1 and then in 10 ml of PBS/0.1% Tween 20 containing 2 drops of Vectastain reagent B only for 30 minutes at 37°C. Subsequent washes and HRP visualisation were carried out as described in Example 1. The results were similar to those obtained in Example 1 with a hybridisation signal corresponding only to pIF55 $alpha_2$ DNA.

The ability of the probe of Examples 1 and 2 to distinguish mismatches is highlighted by the following comparative Example A:-

## Example A

The procedure described in Example 2 was repeated except that the biotinylated oligonucleotide was used directly for hybridisation, Vectastain reagent additions before hybridisation being omitted. After hybridisation, 2 drops of Vectastain reagent A were added to 10 ml. PBS/0.1% Tween 20, prior to Vectastain reagent B addition as described in Example 2. This mixture of Vectastain reagent A and reagent B was incubated at room temperature for ½ hour before use, to allow cross links to form between the avidin (reagent A) and the biotinylated HRP (reagent B). A limit of hybrid detection corresponding to 30 ng pIF $alpha_2$ DNA was obtained compared to 1 ug of pIF55-F6 DNA.

## Example 3

A molecule of biotin was attached to a 15 base long oligonucleotide $alpha_2$ 25 with the nucleotide sequence of (11) by the following method.

### Preparation 1

Using a similar procedure to that described in Preparation 1 of Example 1, but with octane-1,8-diol replacing 2-hydroxypropionitrile, crude biotinyl 2-chlorophenyl 8-hydroxyoctyl phosphate was obtained. The crude mixture was slurried with EtOAc (100 ml.) and filtered to remove excess octane-1,8-diol. The EtOAC extract was evaporated and the residue again slurried with EtOAC (100 ml.) and filtered. After removal of solvents the residue was purified by flash silica gel chromatography (Merck Kieselgel 60 ART 9385) using EtOAc:EtOH (4:1 v/v as eluant. After removal of solvents from appropriate fractions there was thus obtained biotinyl 2-chlorophenyl 8-hydroxyoctyl phosphate (7) in 41% overall yield (molecular ion:$M^+$ 549).

### Preparation 2

Using a similar procedure to that described in Preparation 1 of Example 1 but with biotinyl 2-chlorophenyl 8-hydroxyoctyl phosphate (0.61 g.) replacing biotinol and EtOAc replacing chloroform there was obtained a crude sample of compound (8). This was purified by flash chromatography as described in Preparation 1 to give compound (8) as a colourless oil (300 mg.) (molecular ion: $(M+H)^+$ 792).

### Preparation 3

A solution of compound (8) (280 mg.) in pyridine (3 ml.) and triethylamine (2 ml.) was allowed to stand at 20°C for 6 hours then evaporated. The residual oil was extracted with ether (5 x 20 ml.) and the residue dried by azeotropic distillation with anhydrous pyridine at reduced pressure to give the triethylammonium salt of compound (9).

Preparation 4

The procedure described in Preparation 3 of Example 1 was repeated with oligodeoxyribonucleotide sequence alpha$_2$-25 [M. D. Edge et al. Nucleic Acids Res., 1983, 11, 6419-6435] replacing alpha$_2$-55 and the triethylammonium salt of compound (9) replacing the triethylammonium salt of biotinyl 2-chlorophenyl phosphate to give the biotinylated oligodeoxyribonucleotide sequence (10).

Plasmids for analysis contained either the interferon-alpha$_2$ gene nucleotide sequence 3' CTTTACTAAGTTGTC 5' (M. D. Edge et al., Nucleic Acids Research, 1983, 11, 6419-6435) homologous to the sequence of oligonucleotide (11) or a derivative with the sequence 3' CTTGACTAAGTTGTC 5' which differed by the single underlined residue. This latter plasmid was obtained as follows:-

A gene was prepared from synthetic oligonucleotides essentially as described by M. D. Edge et al - (Nucleic Acids Research, 1983 11, 6419-6435) for synthesis of an interferon-alpha$_2$ gene except that oligonucleotides 1, 2, 24 and 25 were replaced by the sequences 1 Taq, 2 Taq, 24-I4 and 25-I4 respectively.

1 Taq 5' CGACGATGTGTGAT
2 Taq 5' CGGCAGATCACACATCGT
24-I4 5' ATCAGTTCGTGCAGT
25-I4 5' GAACTGATTCAACAG

The synthetic gene was then cloned by the method described in Example 1. The plasmid including the oligonucleotide 25-I4 will hereinafter be referred to as pIF25-I4 alpha$_2$.

Plasmids were "dot-blotted" as described in Example 1.

An avidin-alkaline phosphatase (AP) conjugate was attached to the biotinylated nucleotide as follows. An AP-avidin D conjugate (Vector Laboratories) was reconstituted with water to 20,000 units of enzyme/ml. This solution was diluted 5 fold with 10mM HEPES/ 0.15MNaCl pH7.5 and 1 ml. of this mixture was incubated with 2 ug. of the biotinylated oligonucleotide alpha$_2$-25 (11) for 30 minutes at room temperature. The solution was then passed down a 0.5 ml. ararose-avidin column (Sigma) previously equilibrated with 5 × SSC. The column was washed with 2 ml. 5 × SSC and the pooled eluate subjected to two more cycles of agarose-avidin chromatography. tRNA was added to the final eluate to a concentration of 250 ug./ml. and this solution was used for hybridisation. Filter preparation and hybridisation were carried out as described in Example 1. After hybridisation, filters were washed in a mixture containing 6 × SSC, 0.06% sodium pyrophosphate and 20 mM sodium phosphate pH7 twice at room temperature for 5 minutes and once at 40°C for 3 minutes. Filters were then washed 3 times for 5 mins in 0.1M Tris. HCl pH 7.5, 0.1M NaCl, 2mM $MgCl_2$ and 0.05% Triton X-100. Finally the filters were washed twice for 5 minutes in 0.1M Tris pH9.5, 0.1M NaCl, 5mM $MgCl_2$. For visualisation of hybridised biotinylated oligonucleotide-AP conjugates, filters were incubated in AP substrate mixture prepared as described by Leary et al [Proc. Natl. Acad. Sci. USA, 1983, 80, 4046]. The resulting blue deposits localised to hybridised oligonucleotide-AP conjugates were markedly more intense for immobilised pIF25 alpha$_2$DNA compared to pIF25-I4 alpha$_2$. This discrimination forms the basis for a test to detect DNA sequences with one or more alterations in nucleotide sequence homologous to an oligonucleotide probe.

**Example 4**

The procedure described in Example 3 was repeated except that the biotinylated oligonucleotide was bound with avidin alone for hybridisation, analogous to the procedure described in Example 2. Following hybridisation and washing at temperatures to distinguish mismatches from homologous hybrids, filters were washed for 20 minutes at 37°C in 3% BSA in washing buffer 1 [0.1M Tris HCl (pH 7.5), 0.1M NaCl, 2mM $MgCl_2$ and 0.05% Triton X-100]. Biotinylated AP was subsequently added either as a biotinylated AP polymer (Bethesda Research Laboratories (BRL)) at a concentration of 1 ug./ml. in washing buffer 1 or as biotinylated AP (Vector Laboratories). After 10 minutes filters were washed twice for 2 minutes in washing buffer 1 at room temperature and then twice in washing buffer 2 (0.1M Tris pH9.5, 0.1MNaCl, 5mM $MgCl_2$) prior to incubation in AP substrate mixture as described in Example 3. The visualisation results obtained were comparable with those of Example 3.

The ability of the probes of Examples 3 and 4 to distinguish a single mismatch is highlighted by the following comparative Example B.

**Example B**

The procedure described in Example 4 was repeated except that the biotinylated oligonucleotide was

hybridised prior to the addition of streptavidin. Following hybridisation and washing at temperatures to distinguish mismatches from homologous hybrids, filters were washed as in Example 4 and incubated in streptavidin (Bethesda Research Laboratories) at a concentration of 2 ug./ml. in washing buffer for 10 minutes at room temperature. Following incubation with streptavidin, the filters were washed 3 times for 2 minutes in wash buffer 1 prior to the addition of biotinylated AP polymer (BRL), as described in Example 4. Subsequent washing and detection of AP was carried out as described in Example 4. The visualisation results obtained were comparable with those of Examples 3 and 4.

## Example 5

The procedure described in Preparation 3 of Example 1 was repeated using the triethylammonium salt of (9) instead of the triethylammonium salt of biotinyl-2-chlorophenyl-phosphate to yield the biotinylated oligodeoxyribonucleotide sequence (12).

The hybridisation procedure described in Example 1 was repeated using the biotinylated oligodeoxyribonucleotide sequence (12) as the probe except that the plasmid pIFS1101 (Edge et al., Nucleic Acids Res., 11 (1983) p 6419) was used as a source of the normal interferon $alpha_2$ gene. As observed for Example 1, a hybridisation signal corresponding to 300 ng of pIFS1101 DNA was obtained but was absent from up to 1 ug of the mutant pIF55-F6 $alpha_2$ plasmid DNA.

## Example 6

The procedure described in Example 2 was repeated using the biotinylated oligodeoxyribonucleotide sequence (12) bound with avidin alone for hybridisation to the plasmids pIFS1101 and pIF55-F6 $alpha_2$.

1 ug of pIFS1101 DNA was detectable in this experiment in the absence of signal from 1 ug of pIF55-F6 DNA.

The ability of the probes of Examples 5 and 6 to distinguish mismatches is highlighted by the following comparative Example C:

## Example C

The procedure described in Example A was repeated using the biotinylated oligodeoxyribonucleotide sequence (12) for hybridisation to plasmids pIFS1101 and pIF55-F6 $alpha_2$, hybridization being effected prior to the addition of streptavidin. A minumum of 3 ng of pIFS1101 DNA was detected by this procedure compared to a minimum of 300 ng of pIF55-F6 $alpha_2$ DNA.

## Example 7

The procedure described in Preparation 3 of Example 1 was repeated using the oligodeoxyribonucleotide sequence of $alpha_2$-25(11) [M. D. Edge et al, Nucleic Acids Res. 1983, 11, 6419-6435] instead of the oligodeoxyribonucleotide sequence of $alpha_2$-55(6) to yield the biotinylated oligodeoxyribonucleotide sequence (13).

The hybridisation procedure described in Example 3 was repeated using the biotinylated oligodeoxyribonucleotide sequence (13) as the probe. As shown in Figure 1, a hybridisation signal was observed for the immobilised pIF-25 $alpha_2$ DNA (row B, 3 and 1 ug (left to right) but was absent from the mutant pIF25-I4 $alpha_2$ plasmid DNA (row A, 3 and 1 ug).

## Example 8

A molecule of alkaline phosphatase was covalently linked to a 15 base long oligonucleotide $alpha_2$-55 with the nucleotide sequence of (6) by the following method:-

### Preparation 1

Chloro N,N-diisopropylamino methoxyphosphine was added slowly to a solution of S-trityl-6-mercaptohexanol (2.0 g) and N-ethyl isopropylamine (4.2 g) at 0 °C and the mixture stirred for 15 minutes. Ethyl acetate (100 ml) was then added and the solution extracted with saturated sodium bicarbonate solution (100 ml) then brine (100 ml). The ethyl acetate extract was dried ($MgSO_4$), concentrated to 4.0 ml and applied to a column of silica gel (Kieselgel 60, 12.5 cm $\times$ 5.0 cm) and fractionated using petroleum ether (b.p. 40-60 ° C): triethylamine (9:1 v/v). After evaporation of solvents from combined appropriate fractions N,N-diisopropylamino methoxy S-trityl-6-mercaptohexoxy-phosphine (14) was obtained.

Preparation 2

The fully protected oligodeoxyribonucleotide sequence alpha$_2$-55 was prepared on an Applied Biosystems 380A DNA synthesiser from 5'-dimethoxytrityl-N$^2$-isobutyryl-2'-deoxyguanosine bound to Fractosil via 3'-OH and a succinylglycylglycylaminopropyl spacer (BDH Chemicals Ltd) and 2-cyanoethyl-N,N-diisopropylamino phosphoramidites of 5'-dimethoxytrityl-N$^6$-benzoyl-2'-deoxyadenosine, 5'-dimethoxytrityl-N$^4$-benzoyl-2'-deoxycytidine, 5'-dimethoxytrityl-N$^2$-isobutyryl-2'-deoxyguanosine and 5'-dimethoxytritylthymidine (BDH Chemicals Ltd). Alternatively, the fully-protected oligodeoxyribonucleotide sequence may be prepared by the manual methods as described by Atkinson and Smith in 'Oligonucleotide Synthesis, a Practical Approach'. (M J Gait Editor, IRL Press, Oxford, Washington DC pp 35-81).

N,N-Diisopropylamino methoxy S-trityl-6-mercaptohexoxyphosphine (53.7 mg) was dried by azeotropic distillation with anhydrous acetonitrile (3 × 1 ml) then dissolved in an anhydrous mixture of 1,2-dichloroethane: acetonitrile (3:2 v/v (1.5 ml). This solution was used in place of a normal nucleoside phosphoramidite in a standard coupling reaction on an Applied Biosystems 380A DNA synthesiser and added to the 5'-end of the fully-protected oligodeoxyribonucleotide sequence alpha$_2$-55 described above. The procedures briefly consisted of : (1) removal of the dimethoxytrityl group with 3% trichloroacetic acid in 1,2-dichloroethane; (2) coupling of the N,N-diisopropylamino methoxy S-trityl-6-mercaptohexoxyphosphine (5.37 mg in 0.15 ml of solvent) after activation with tetrazole; (3) iodine oxidation of the intermediate phosphite to a phosphate; (4) a capping step with acetic anhydride. Partial deprotection and cleavage of the oligonucleotide sequence from the Fractosil support was performed automatically on the DNA synthesiser. Phosphate protecting groups were removed by treatment with thiophenolate then with ammonium hydroxide (d.0.88) which also cleaves the oligodeoxyribonucleotide sequence from the support. The ammonium hydroxide solution was heated at 55 ° C for 6 hours then evaporated and the residue dissolved in ethanol/water (3:7 /v/ 1.5 ml). The trityl-protected sequence (15) was purified initially by hplc on Partisil 10-SAX ion exchange resin as described in Preparation 3 of Example 1. Further purification was achieved using reverse-phase hplc on a C18-u Bondapak column as described by C R Newton et al, (Anal. Biochem. 1983 129, 22-30) except that a gradient from 5% acetonitrile in 0.1M ammonium acetate to 50% acetonitrile in 0.1M ammonium acetate over 40 minutes replaced the gradient of 5% to 12½% acetonitrile in 0.1M ammonium acetate.

Preparation 3

Compound (15) (40 pmoles) was$^{32}$P-labelled with 2',3'-dideoxyadenosine-5'-[alpha-$^{32}$P]triphosphate using a 3'-end labelling kit (Amersham International PLC) as instructed by the manufacturer except that a mixture of 2',3'-dideoxyadenosine-5'-triphosphate and the [alpha-$^{32}$P]derivative ( 4 : 1 ) was used. The labelled oligodeoxyribonucleotide was purified on a NENSORB 20 cartridge (DuPont/NEN Research Products) as instructed by the manufacturer.

Silver nitrate (61 nmoles) in water (1.25 ml) was added to a solution of (15) (1.5 nmoles) and the 3'-end labelled (15) (0.1 nmoles) in water (246 µl). After 30 minutes at 20 ° C a solution of dithiothreitol (63 nmoles) in water (1.25 ml) was added. After a further 30 minutes at 20 ° C the mixture was filtered through a Millex-GS filter unit (0.22 µm) and evaporated to a volume of 250 µl to give a solution of compound (16) (1.5 nmoles) and the $^{32}$P 3'-end labelled derivative (0.01 nmoles).

Preparation 4

A solution of m-maleimidobenzoyl-N-hydroxysuccinimide ester (MBS) (0.12 mg) in dimethylformamide (DMF) (5.0 µl) was added to a solution of calf intestine alkaline phosphatase (Boehringer Mannheim 567752) (3.0 mg) in 0.05M phosphate buffer, pH 7.0 (0.7 ml). The mixture was stirred at 20 ° C for 30 minutes then applied to a Sephadex G-25 column (5 × 1.5 cm) equilibrated with 0.1M phosphate buffer, pH 5.6 and eluted with the same buffer at 4 ° C. Fractions (0.5 ml) were collected and monitored for UV absorbance at 280 nm to give compound (17).

Preparation 5

The solution of (16) (250 µl) containing the tracer amount of $^{32}$P3'-end labelled derivative (from

Preparation 3) was added to the solution of derivatised alkaline phosphatase (17) (from Preparation 4). After 1 hour at 20°C the solution was applied to a Sephadex G-50 column (26 cm × 1.0 cm) equilibrated with 0.1M phosphate buffer; pH5.6) and eluted at 0.9 ml min $^{-1}$ with the same buffer. Fractions (1 ml) were collected and monitored by scintillation counting and UV absorbance at 280 nm. Fractions containing both the alkaline phosphatase (UV absorbance at 280 nm) and $^{32}$P-radioactive label thus contain the alpha$_2$-55 oligonucleotide-alkaline phosphatase complex (18).

45 pmoles of alpha$_2$-55 oligonucleotide alkaline phosphatase complex in 0.88 ml of 0.1M sodium phosphate buffer at pH5.6 was concentrated to 0.25 ml in the same buffer as follows:
BSA (Fraction V, Miles) was added to 1 ug/ml and 2 equal aliquots of the sample were loaded onto a Centricon centrifugal microconcentrator (Amicon Ltd., Stonehouse, UK). The samples were concentrated by spinning the devices in a Sorvall SS34 rotor at 1000 g for 30 minutes.

As described in Example 1, serially diluted samples of either pIF55 alpha$_2$ or pIF55-F6 alpha$_2$ plasmid were "dot-blotted" onto nitrocellulose. Prehybridisations were also as described in Example 1. The hybridisation solution consisted of 2 ml of 5 × SSC, 350 ug/ml tRNA containing, 45 pmoles of oligonucleotide-alkaline phosphatase complex (18) in 250 ul buffer.

Following an overnight hybridisation at room temperature, filters were washed twice for 5 minutes at room temperature in a mixture containing 6 × SSC, 0.06% sodium pyrophosphate and 20mM sodium phosphate, pH7.0. Filters were then further washed in the above mentioned buffer at 39°C for 2 minutes under which conditions the oligonucleotide complex hybridised to the interferon alpha$_2$ gene sequence with three terminal mismatches was washed off the filter to a much greater extent than the oligonucleotide complex which had formed a perfect hybrid with the interferon-alpha$_2$ gene. The filters were further washed three times for 5 minutes in 0.1M Tris. HCl pH7.5, 0.1M NaCl, 2mMMgCl$_2$ and 0.05% Triton-X-100. Finally the filters were washed twice for 5 minutes in 0.1M Tris,pH9.5, 0.1M NaCl, 5mMMgCl$_2$ and subsequently incubated with the AP substrate mixture as described in Example 3. Figure 2 shows the result of this experiment and indicates discrimination by the oligonucleotide-alkaline phosphatase complex between (left to right) 300 ng and 30 ng of the immobilised pIF55alpha$_2$ plasmid DNA (row A) and the pIF55-F6alpha$_2$ derivative plasmid DNA (row B) which has a three base pair deviation from the interferon alpha$_2$ gene (see Example 1). The pIF55alpha$_2$ plasmid DNA (row C) and its derivative pIF55-F6alpha$_2$ (row D), were also mock hybridised without the oligonucleotide complex but with 14 nmoles of alkaline phosphatase (Boehringer, code no 567752) included in 2 ml of hybridisation buffer as specified above. No marked signal was seen associated with immobilised DNA in rows C and D thus ruling out artefacts due to direct binding of alkaline phosphatase.

## Example 9

A molecule of alkaline phosphatase was covalently attached to a 15 base long oligodeoxyribonucleotide alpha$_2$-55F6 with the nucleotide sequence of (20) by the procedure described in Preparations 2,3,4 and 5 of Example 8 with the following modifications:

1) Preparation 2

The fully-protected oligodeoxyribonucleotide alpha$_2$55F6 with the nucleotide sequence (20) was prepared on an Applied Biosystems 380A DNA synthesiser from 5'-dimethoxytrityl-N$^4$-benzoyl-2'-deoxycytidine bound to controlled pore glass (Pore size 500A; Particle size 125-174 micron; loading 20-50 micromol/g from Cruachan Chemical Co Ltd.). N,N-diisopropylamino methoxy S-trityl-6-mercaptohexoxyphosphine was coupled to the partially deprotected oligodeoxyribonucleotide bound to controlled-pore glass (step 2) by 2 additions, each of 100 second duration, of the tetrazole activated phosphine. The capping step (step 4) was omitted. The ammonium hydroxide solution containing the oligodeoxyribonucleotide after cleavage from the support was heated at 50°C for 4 hours.

2) Preparation 3

The amounts of silver nitrate and dithiothreitol were increased to 120 nmoles and 122 nmoles respectively.

3) Preparation 4

The amount of MBS was reduced to 0.08 mg in DMF (3.4 ul) and the alkaline phosphatase reduced to

2.0 mg in 0.05M phosphate buffer, pH 7.5 (0.23 ml).

4) Preparation 5

The reaction was carried out for 1 hour at 37° C and the column was equilibrated with 0.1M phosphate buffer at pH 5.0.

Using this procedure the alpha$_2$-55F6 oligonucleotide alkaline phosphatase complex (19) was obtained.

The alpha$_2$-55F6 oligonucleotide-alkaline phosphatase complex was directly hybridised to dilutions of immobilised pIF55alpha$_2$ or pIF55-F6alpha$_2$ DNA. Prehybridisation, hybridisation and washing conditions were as described for Example 8 except that the hybridisation was conducted in 10 ml of buffer including 120 pmoles of alpha$_2$-55 F6 oligonucleotide-AP complex (19) in 0.91 ml 0.1M sodium phosphate, pH5.0. Figure 3 shows the results of this experiment and again indicates discrimination by the oligonucleotide-AP complex between (left to right) 300 ng and 30 ng of pIF55 alpha$_2$ (row A) and pIF55-F6alpha$_2$ (row B), signal intensity produced by the probe being stronger for the homologous plasmid pIF55-F6alpha$_2$.

**Example 10**

The procedure described in Example 9 was repeated with oligodeoxyribonucleotide sequence (11) replacing the oligodeoxyribonucleotide sequence (20). In Preparation 2 of Example 9 the oligodeoxyribonucleotide was prepared from 5'-dimethoxytrityl-N$^2$-isobutyryl-2'-deoxyguanosine bound to controlled-pore glass. Preparation 3 was carried out as described in Preparation 3 of Example 8.

As described in Example 8 serially diluted samples of either pIF25alpha$_2$ or pIF25-14alpha$_2$ DNA were immobilised onto nitrocellulose filters. The filters were subsequently prehybridised, hybridised and washed as described in example 9 except that hybridisation was conducted in 10 ml ofbuffer including 170 pmoles of alpha$_2$-25 oligonucleotide-AP comlex (21) in 1 ml 0.1M sodium phosphate, pH5.6. Figure 4 shows the results of this experiment indicating much stronger hybridisation of the probe to (left to right) 300 ng, 30 ng and 3 ng of the homologous plasmid pIF25alpha$_2$ (row A) than to 300 ng, 30 ng and 3 ng the plasmid pIF25-14alpha$_2$ (row B) which had a single base non-complementary to the oligonucleotide probe.

**Example 11**

The procedure described in Example 8 was repeated except that hybridisation included 45 pmoles of alpha$_2$-55 oligonucleotide-alkaline phosphatase complex (18) in 0.4 ml buffer, 0.025% BSA, 5 × SSC and 250 µg/ml tRNA in 4 ml total volume. Following hybridisation, filters were washed as in example 9 except that the final 5 minute washes in 0.1M Tris pH9.5, 0.1MNaCl and 5 nM $MgCl_2$ were replaced by a 5 minute wash at room temperature in washing buffer (working strength) from an Ampak enzyme amplification kit (IQ (Bio) Ltd., Cambridge, UK). A further 5 minute wash in 0.1M Tris pH9.5, 0.1MNaCl and 5mM$MgCl_2$ at room temperature was followed by 5 sequential room temperature washes of 5 minutes each in Ampak washing buffer concentrate. Finally individual immobilised DNA samples were cut out of the filters and placed in individual wells of a microtitre dish (24 well, Falcon 3047). To each sample was added 100 µl reconstituted Ampak Substrate mixture and incubated at room temperature for 20 minutes. 200 µl reconstituted Ampak Amplifier mixture was added and the solution left for 10 minutes for slow colour development before adding 50 µl stopping solution per well. Absorbances were then read at 495 nm. There was a clear increase in absorbance for the oligonucleotide complex hybridised to the pIF55alpha$_2$ plasmid DNA compared to the derivative plasmid pIF55-F6 alpha$_2$ DNA. For example, 30 ng of the pIF55 alpha$_2$ DNA gave rise to an $OD_{495}$ of approximately 0.7 units above background while the signal from pIF55-F6 alpha$_2$ was not significantly above background.

Example 12

The oligonucleotide (alpha$_2$55)-alkaline phosphatase complex (18), as described in example 9, was used for hybridisation to a plasmid DNA/human DNA mixture after Southern blot transfer to nitrocellulose. DNA was extracted from human Daudi Burkitt Lymphoma cells by the method of Blin and Stafford (Nucleic Acids Research, Volume 3 (1976) page 2303) and was dissolved at 1 mg/ml in 10mM Tris HCl pH8, 1mM EDTA. To 200 ul of this DNA solution was added 120 µl $H_2O$, 40 µl of 10 × Core Buffer (Bethesda Research Laboratories) and 40 ul EcoR1 (Cambridge Biotechnology Laboratories, Cambridge UK; 20 µ/µl). The solution was incubated at 37° C for 3 hours and then extracted once with phenol/chloroform (1:1), once with chloroform and three times with ether. DNA was then precipitated by addition of sodium acetate to 0.3M

and 2 volumes of ethanol. The pelletted DNA was washed in 70% ethanol and redissolved in 200 µl $H_2O$.

1.5 µg of plasmids containing either the interferon-$alpha_2$ gene (pIF55$alpha_2$) or its derivative including the oligonucleotide $alpha_2$-55 (pIF55-F6 $alpha_2$) were similarly digested with EcoRI using 20 units for pIF55$alpha_2$ and 40 units for pIF55-F6$alpha_2$ in volumes of 10 µl and 20µl respectively. DNA was solvent extracted and ethanol precipitated as above and finally dissolved in 15 µl $H_2O$.

10 µg samples of Daudi cell DNA were mixed with either 300 ng and 30 ng of pIF55 $alpha_2$ or pIF55-F6 $alpha_2$ DNA and electrophoresed in a 1% agarose gel as described by Maniatis et al. (Molecular Cloning ; A Laboratory Manual: Cold Spring Harbor Laboratory, 1982). DNA was then transferred to nitrocellulose using the Southern transfer procedure as described in Maniatis et al (see above). The filter was air dried and baked in vacuo for 2 hours at 80° C. Details of the prehybridisation, hybridisation and filter washing conditions were as described in Example 8 except that hybridisation was performed in 8 ml of a mixture of 95 pmoles of $alpha_2$-55 olignonucleotide-alkaline phosphatase, 5 × SSC, 250 ug/ml tRNA, 0.05% BSA and 0.6 mM sodium phosphate pH 5.6. Figure 5 shows the results of this analysis indicating that 300 ng of linearised plasmid pIF55 $alpha_2$ could be detected as a single band either in the presence (lane 1) or absence (lane 5) of an excess of human DNA. Under the same conditions , 300 ng of linearised pIF55-F6$alpha_2$ DNA was not detectable in the presence (lane 2) or absence (lane 6) of Daudi DNA demonstrating discrimination by the $alpha_2^{55}$ oligonucleotide-AP complex (18) between pIF55$alpha_2$ and pIF55-F6$alpha_2$ following the Southern transfer procedure.

Example 13

The oligonucleotide $alpha_2$ 25-AP complex (21) as described in example 10 was used for hybridisation to a plasmid DNA/human DNA mixture after Southern blot transfer to nitrocellulose. The plasmid DNA's utilised in the experiment were pIF25 $alpha_2$ and pIF25-I4 $alpha_2$ digested as described for pIF55 $alpha_2$ and pIF55-F6 $alpha_2$ respectively in Example 12. Samples of EcoRI digested plasmid DNA was mixed with EcoRI digested Daudi cell DNA and subjected to electrophoresis as in Example 12. Prehybridisation, hybridisation, washing and oligonucleotide-AP detection conditions were as in Example 10. As observed for example 12, aliquots of pIF25 $alpha_2$ plasmid DNA, homologous to the probe, could be detected as a single band to an equal extent in the presence or absence of Daudi cell DNA. Under these same conditions, no signal corresponding to the plasmid pIF25-I4$alpha_2$ either in the presence or absence of Daudi cell DNA was detectable demonstrating discrimination, following Southern transfer, by the $alpha_2$ 25 oligonucleotide-AP probe (21) between the homologous plasmid, pIF25 $alpha_2$, and the plasmid pIF25-I4 $alpha_2$ which has a single base non-complementary to the probe.

In the following formulae drawings Z represents an oligonucleotide and AP represents alkaline phosphatase. Throughout the specification the terminal phosphate of an oligonucleotide is considered to be part of the oligonucleotide, but in the drawings the terminal phosphate has been shown separately so as to illustrate the reactions which occur on this phosphate moiety.

Formulae

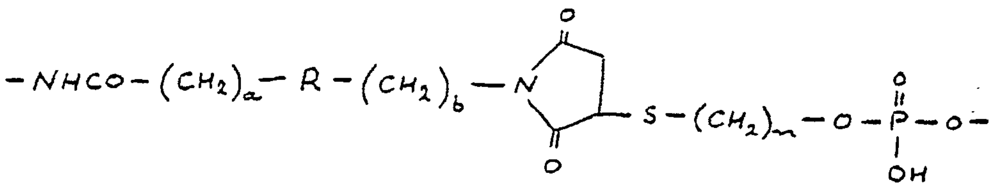

(A)

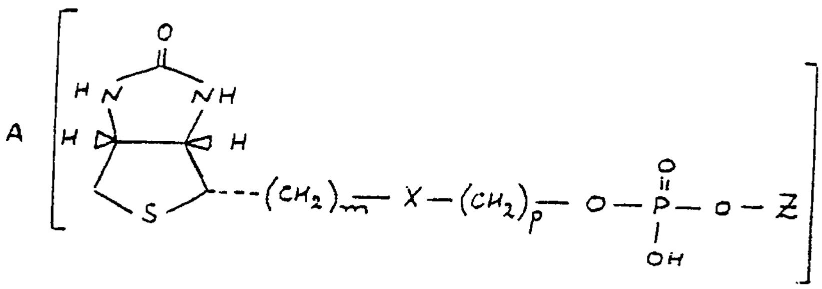

(B)

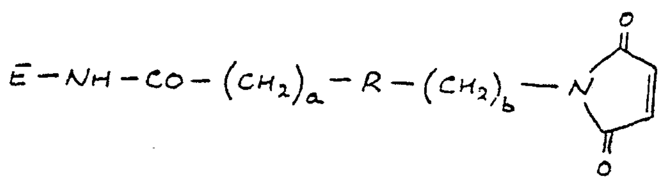

(C)

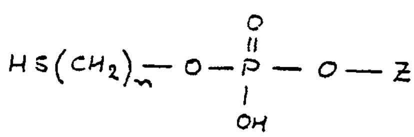

(D)

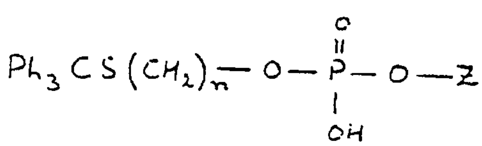

(E)

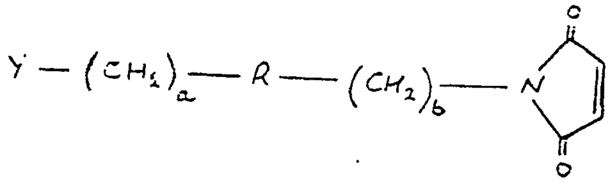

(F)

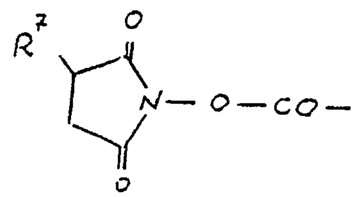

(G)

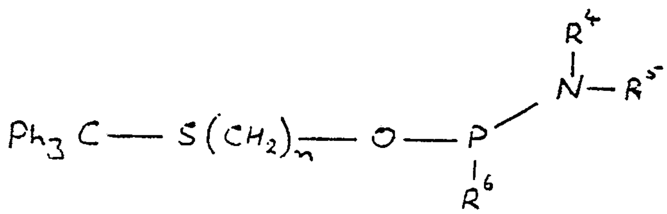

(H)

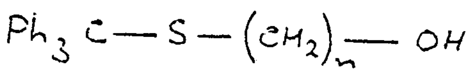

(J)

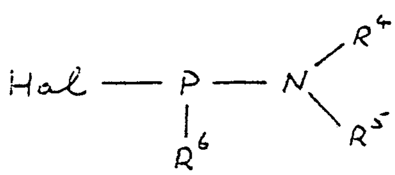

(K)

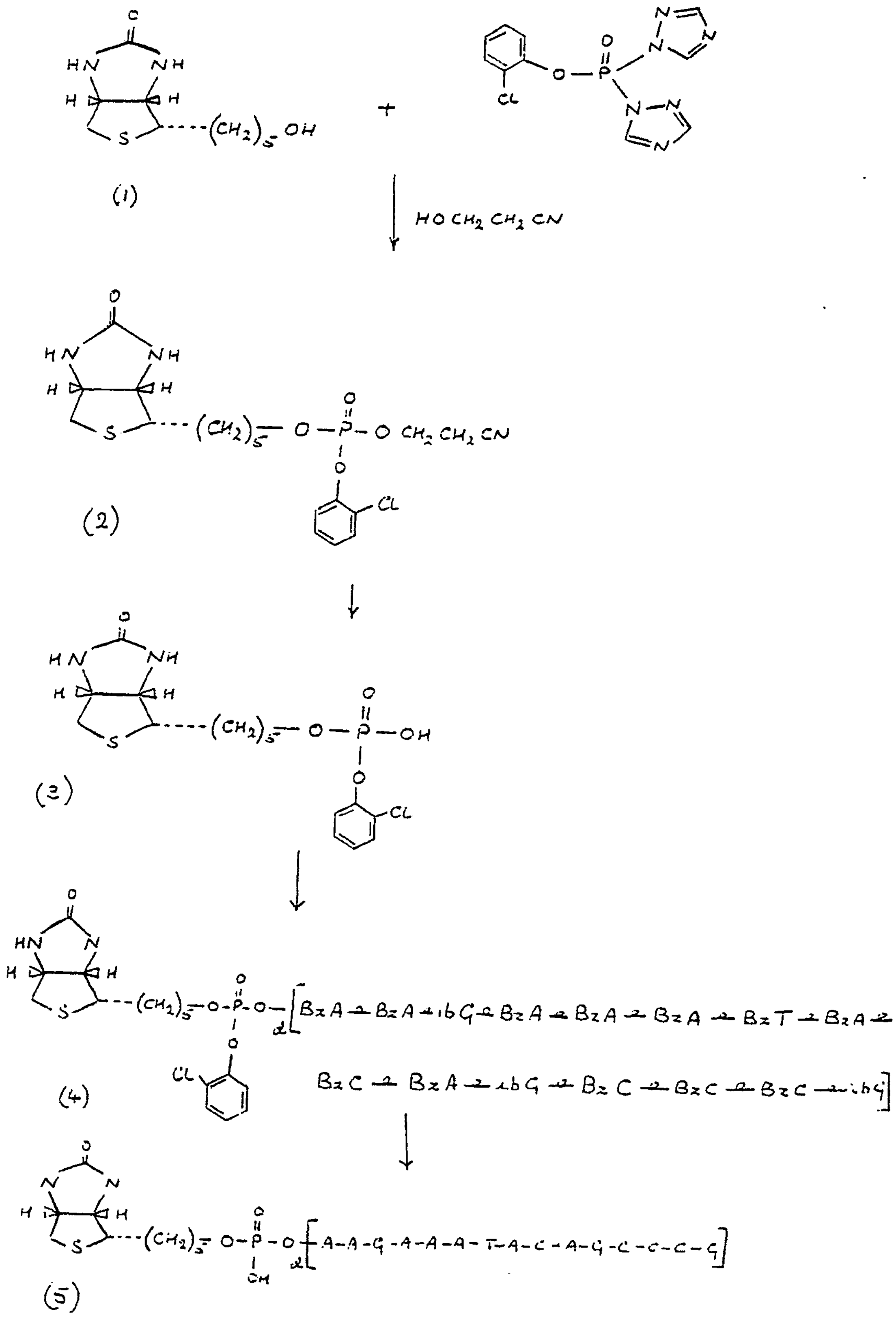


(1)

(2)

(3)

(4)

(5)

d[A-A-G-A-A-A-T-A-C-A-G-C-C-C-G] (6)

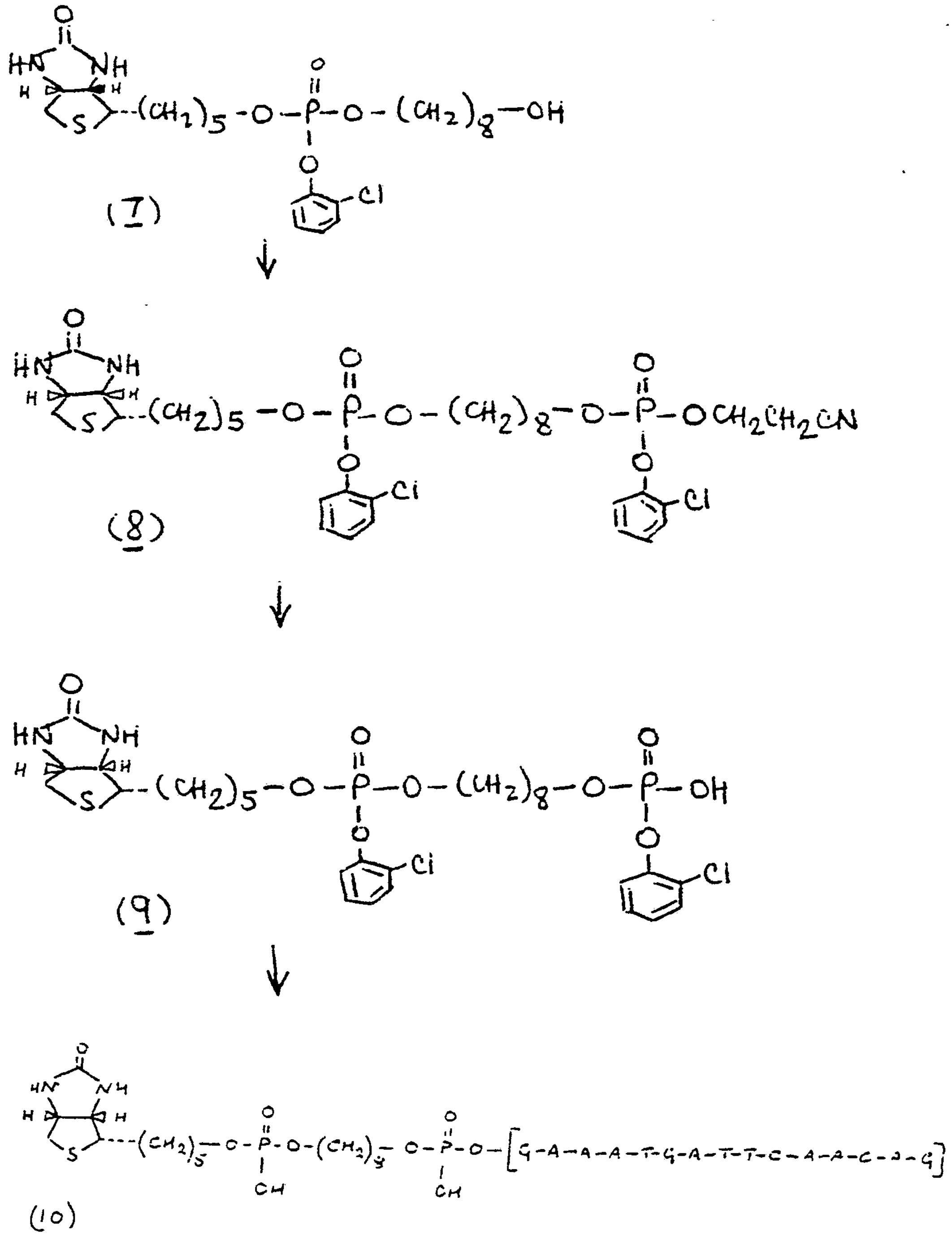

(7)

(8)

(9)

(10)

d[G-A-A-A-T-G-A-T-T-C-A-A-C-A-G] (11)

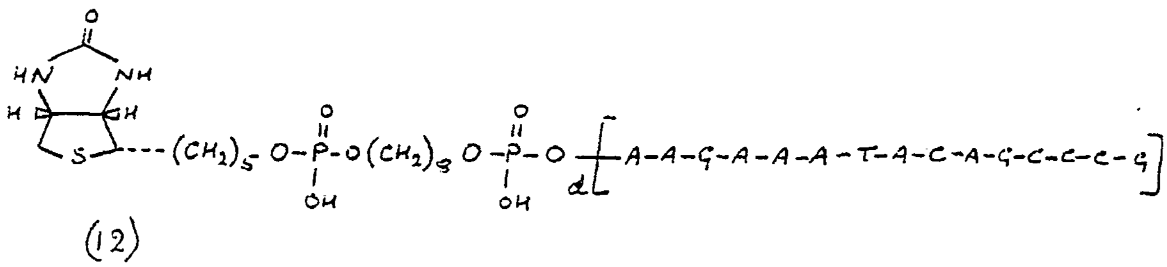

(12)

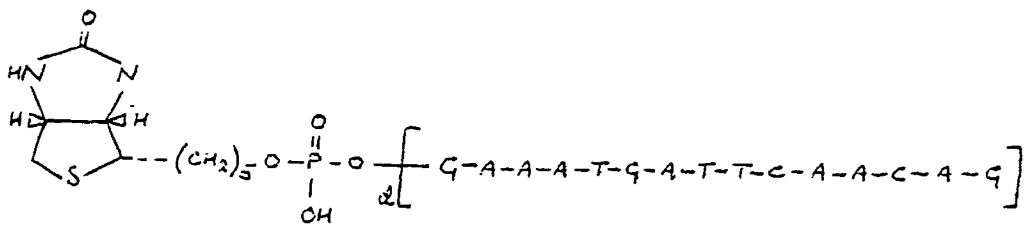 (13)

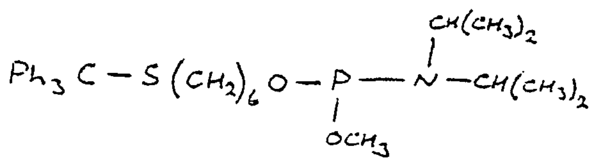 (14)

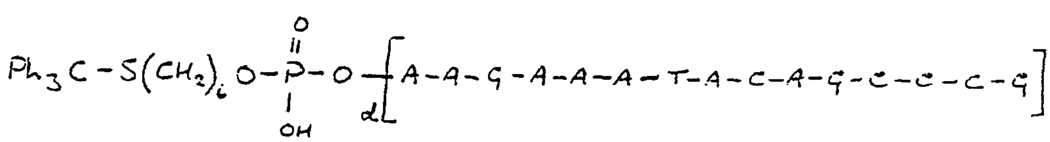

(15)

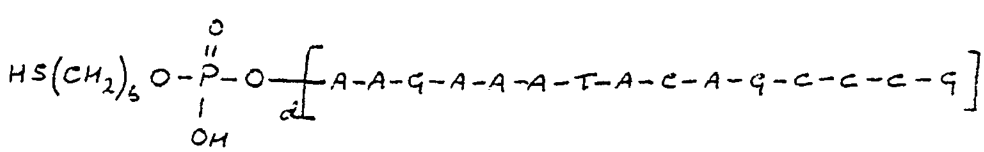

(16)

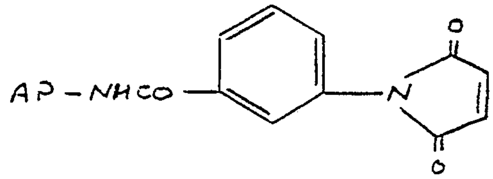

(17)

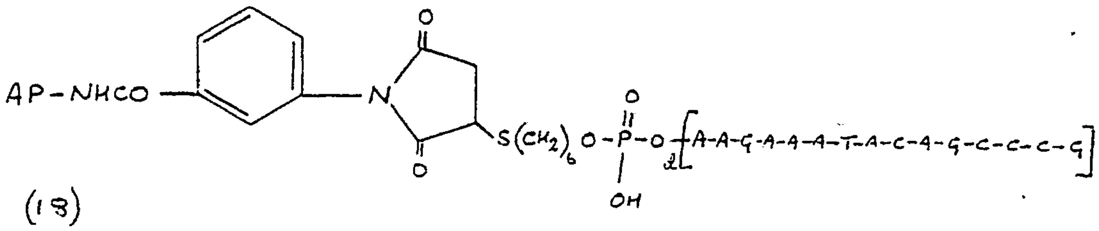

(18)

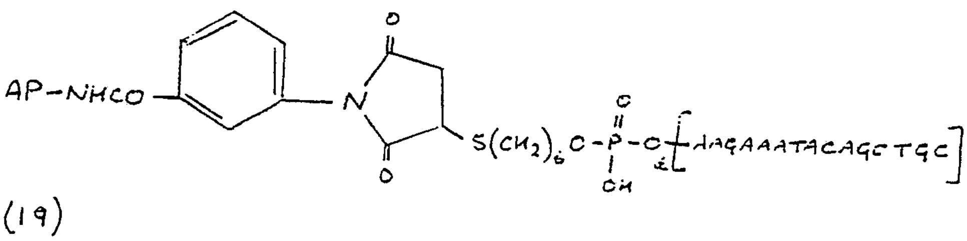

(19)

d[A-A-G-A-A-A-T-A-C-A-G-C-T-G-C] (20)

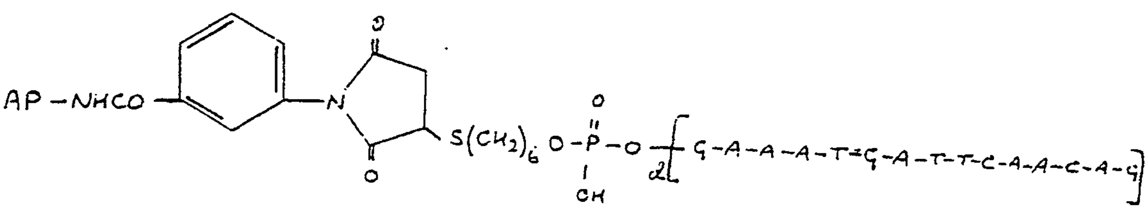

(21)

## Claims

1. A method for discriminating between two nucleotide sequences differing by as little as 1-3 nucleotides which comprises:-

(a) under conditions which promote or favour hybridisation between two complementary oligonucleotide strands, contacting the nucleotide sequence (after treatment to render it single stranded) to be identified or determined with an oligonucleotide signalling complex probe comprising a single stranded oligonucleotide of 8 to 30 nucleotide units in length and having covalently attached thereto, at a sterically tolerant site, a residue having a molecular weight of at least 1,000 daltons, which residue is capable of producing a non-radioisotopic signal, the oligonucleotide in the oligonucleotide-signal complex having a defined sequence which is complementary to a sequence to

be identified or determined but which is not complementary to an alternative sequence;
(b) separating the probe:test nucleic acid hybrid from the unhybridized probe; and
(c) producing or observing the signal resulting from the hybridised oligonucleotide-signalling complex.

2. A method as claimed in claim 1 wherein the residue has a molecular weight of at least 5000 daltons.

3. A method as claimed in claim 2 wherein the residue has a molecular weight of at least 20,000 daltons.

4. A method as claimed in any one of the preceding claims effective to discriminate between two complementary nucleotide sequences differing by as little as a single nucleotide.

5. A method as claimed in any one of the preceding claims wherein the residue which is capable of producing a non- radioisotopic signal comprises a proteinaceous moiety.

6. A method as claimed in any one of the preceding claims wherein the oligonucleotide signalling complex probe comprises a single stranded oligonucleotide sequence having a detection system covalently attached thereto via a spacer group, said detection system comprising a system for producing an enzymatically activated production of colour change, the system being linked to the spacer group by a direct covalent link or by a specific binding pair.

7. A method as claimed in any one of the preceding claims wherein the oligonucleotide signalling complex comprises a single stranded oligonucleotide of 8 to 30 nucleotide units in length having an enzyme label linked to the oligonucleotide via a spacer group, the linkage of the spacer group to the oligonucleotide being via the terminal base, sugar or phosphate.

8. A diagnostic kit for discriminating between two complementary nucleotide sequences differing by as little as a single nucleotide, one of the said sequences being associated with a disease state and the other of said sequences being the corresponding normal sequence, which kit comprises 1) at least one oligonucleotide signalling complex probe comprising a single stranded oligonucleotide of 8 to 30 nucleotide units in length having a sequence complementary to a sequence associated with a disease state and 2) at least one oligonucleotide signalling complex probe comprising a single stranded oligonucleotide of 8-30 nucleotide units in length having a sequence complementary to the corresponding normal sequence which would be present in a human not subject to the said disease state, said oligonucleotide having covalently attached thereto, at a sterically tolerant site, a residue having a molecular weight of at least 1000 daltons, which residue is capable of producing a non-radioisotopic signal.

**Revendications**

1. Procédé pour différencier deux séquences de nucléotides, dont la différence peut être limitée à 1-3 nucléotides, qui consiste :
(a) dans des conditions qui activent ou favorisent l'hybridation entre deux brins oligonucléotidiques complémentaires, à mettre en contact la séquence de nucléotides (après traitement pour la rendre monocaténaire), à identifier ou déterminer, avec une sonde complexe de signalisation d'oligonucléotide comprenant un oligonucléotide monocaténaire long de 8 à 30 motifs nucléotidiques et auquel est fixé par covalence, à un site stériquement tolérant, un résidu ayant un poids moléculaire d'au moins 1000 daltons, résidu qui est capable d'engendrer un signal non radio-isotopique, l'oligonucléotide dans le complexe de signalisation d'oligonucléotide ayant une séquence définie qui est complémentaire d'une séquence à identifier ou déterminer, mais qui est non complémentaire d'une autre séquence ;
(b) à séparer l'hybride sonde:acide nucléique à tester de la sonde non hybridée ; et
(c) à engendrer ou observer le signal résultant de la présence du complexe de signalisation d'oligonucléotide hybridé.

2. Procédé suivant la revendication 1, dans lequel le résidu possède un poids moléculaire d'au moins 5000 daltons.

3. Procédé suivant la revendication 2, dans lequel le résidu possède un poids moléculaire d'au moins 20 000 daltons.

4. Procédé suivant l'une quelconque des revendications précédentes, efficace pour différencier deux séquences de nucléotides complémentaires, dont la différence peut être limitée à un seul nucléotide.

5. Procédé suivant l'une quelconque des revendications précédentes, dans lequel le résidu qui est capable d'engendrer un signal non radio-isotopique comprend un groupement protéinique.

6. Procédé suivant l'une quelconque des revendications précédentes, dans lequel la sonde complexe de signalisation d'oligonucléotide comprend une séquence oligonucléotidique monocaténaire à laquelle est fixé par covalence un système de détection par l'intermédiaire d'un groupe intercalaire, ledit système de détection comprenant un système destiné à produire un changement de couleur activé par voie enzymatique, le système étant lié au groupe intercalaire par une liaison covalente directe ou par une paire à liaison spécifique.

7. Procédé suivant l'une quelconque des revendications précédentes, dans lequel le complexe de signalisation d'oligonucléotide comprend un oligonucléotide monocaténaire long de 8 à 30 motifs nucléotidiques auquel est lié un marqueur enzymatique par l'intermédiaire d'un groupe intercalaire, la liaison du groupe intercalaire à l'oligonucléotide s'effectuant par l'intermédiaire de la base, du sucre ou du phosphate terminal.

8. Kit de diagnostic pour différencier deux séquences de nucléotides complémentaires, dont la différence peut être limitée à un seul nucléotide, l'une desdites séquences étant liée à un état pathologique et l'autre desdites séquences étant la séquence normale correspondante, kit qui comprend 1) au moins une sonde complexe de signalisation d'oligonucléotide comprenant un oligonucléotide monocaténaire long de 8 à 30 motifs nucléotidiques ayant une séquence complémentaire d'une séquence liée à un état pathologique, et 2) au moins une sonde complexe de signalisation d'oligonucléotide comprenant un oligonucléotide monocaténaire long de 8 à 30 motifs nucléotidiques ayant une séquence complémentaire de la séquence normale correspondante qui serait présente chez un sujet humain ne présentant pas ledit état pathologique, oligonucléotide auquel est fixé par covalence, à un site stériquement tolérant, un résidu ayant un poids moléculaire d'au moins 1000 daltons, résidu qui est capable d'engendrer un signal non radio-isotopique.

## Patentansprüche

1. Ein Verfahren zum Unterscheiden zwischen zwei Nucleotidsequenzen, selbst wenn sich diese nur um 1 bis 3 Nucleotide unterscheiden, das umfaßt:
   (a) unter Bedingungen, die die Hybridisierung zwischen zwei komplementären Oligonucleotidsträngen fördern oder begünstigen, Umsetzen der zu identifizierenden oder zu bestimmenden Nucleotidsequenz (nach einer Behandlung, um diese einzelsträngig zu machen) mit einer Oligonucleotid-Signalkomplexsonde, die ein einzelsträngiges Oligonucleotid umfaßt, das über seine Länge 8 bis 30 Nucleotideinheiten aufweist und daran kovalent angefügt, und zwar an einer sterisch toleranten Stelle, einen Rest aufweist, der ein Molekulargewicht von wenigstens 1000 Dalton aufweist, wobei dieser Rest in der Lage ist, ein Nicht-Radioisotopen-Signal zu erzeugen und wobei das Oligonucleotid in dem Oligonucleotid-Signalkomplex eine definierte Sequenz aufweist, die komplementär zu einer zu identifizierenden oder zu bestimmenden Sequenz ist, die jedoch nicht komplementär zu einer alternativen Sequenz ist;
   (b) Abtrennen des Sonden:Testnucleinsäuren-Hybrids von der unhybridisierten Sonde; und
   (c) Erzeugen oder Beobachten des Signals, das von dem hybridisierten Oligonucleotid-Signalkomplex stammt.

2. Ein Verfahren nach Anspruch 1, worin der Rest ein Molekulargewicht von wenigstens 5000 Dalton aufweist.

3. Ein Verfahren nach Anspruch 2, worin der Rest ein Molekulargewicht von wenigstens 20 000 Dalton aufweist.

4. Ein Verfahren nach irgendeinem der vorausgehenden Ansprüche, das in der Lage ist, zwischen zwei komplementären Nucleotidsequenzen zu unterscheiden, die sich um nur ein einziges Nucleotid unterscheiden.

5. Ein Verfahren nach irgendeinem der vorausgehenden Ansprüche, worin der Rest, der in der Lage ist, ein Nicht-Radioisotopen-Signal zu erzeugen, eine proteinische Einheit umfaßt.

6. Ein Verfahren nach irgendeinem der vorausgehenden Ansprüche, worin die Oligonucleotid-Signalkomplexsonde eine einzelsträngige Oligonucleotidsequenz umfaßt, die ein Nachweissystem aufweist, das über eine Abstandshaltergruppe kovalent an diese angefügt ist, wobei das genannte Nachweissystem ein System zur Erzeugung einer enzymatisch aktivierten Erzeugung einer Farbveränderung umfaßt, wobei das System mit der Abstandshaltergruppe durch eine direkte kovalente Bindung oder über ein spezifisches Bindungspaar verknüpft ist.

7. Ein Verfahren nach irgendeinem der vorausgehenden Ansprüche, worin der Oligonucleotid-Signalkomplex ein einzelsträngiges Oligonucleotid umfaßt, das über seine Länge 8 bis 30 Nucleotideinheiten aufweist und bei dem an das Oligonucleotid über eine Abstandshaltergruppe ein Enzymlabel gebunden ist, wobei die Verknüpfung der Abstandshaltergruppe mit dem Oligonucleotid über die terminale Base, den terminalen Zucker oder das terminale Phosphat erfolgt.

8. Ein diagnostischer Kit zum Unterscheiden zwischen zwei komplementären Nucleotidsequenzen, selbst wenn sich diese nur um ein einziges Nucleotid unterscheiden, wobei eine der genannten Sequenzen mit einem Krankheitszustand assoziiert ist und die andere dieser Sequenzen die entsprechende normale Sequenz darstellt, wobei der Kit aufweist 1) wenigstens eine Oligonucleotid-Signalkomplexsonde mit einem einzelsträngigen Oligonucleotid, das über seine Länge 8 bis 30 Nucleotideinheiten aufweist und eine Sequenz aufweist, die komplementär zu einer Sequenz ist, die mit einem Krankheitszustand assoziiert ist, und 2) wenigstens eine Oligonucleotid-Signalkomplex-Sonde mit einem einzelsträngigen Oligonucleotid, das über seine Länge 8 bis 30 Nucleotideinheiten aufweist und eine Sequenz aufweist, die zu der entsprechenden normalen Sequenz komplementär ist, die in einem Menschen anzutreffen wäre, der sich nicht im Zustand der genannten Krankheit befindet, wobei dieses Oligonucleotid kovalent daran angefügt, und zwar an einer sterisch toleranten Stelle, einen Rest mit einem Molekulargewicht von wenigstens 1000 Dalton aufweist, wobei dieser Rest in der Lage ist, ein Nicht-Radioisotopen-Signal zu erzeugen.

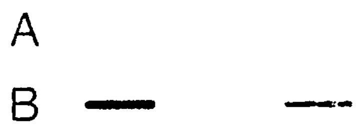


FIG.1

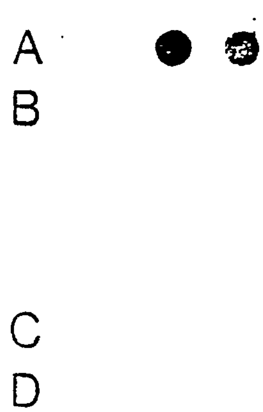


FIG.2

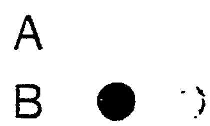


FIG.3

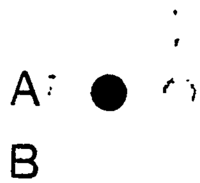


FIG.4

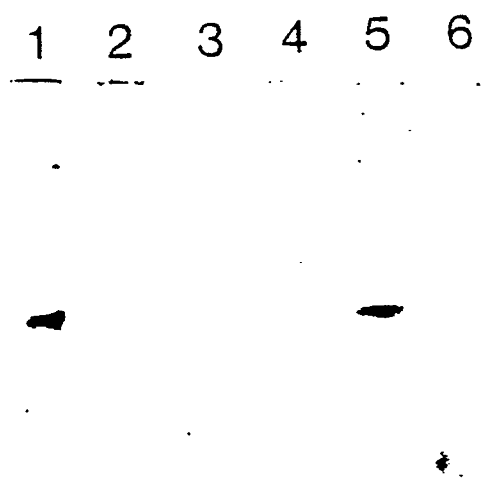


FIG.5